(19) Europäisches Patentamt
European Patent Office
Office européen des brevets

(11) EP 0 643 961 B1

(12) **FASCICULE DE BREVET EUROPEEN**

(45) Date de publication et mention de la délivrance du brevet:
**22.10.1997 Bulletin 1997/43**

(51) Int. Cl.[6]: **A61K 7/48**, A61K 7/06, A61K 7/50

(21) Numéro de dépôt: **94402041.1**

(22) Date de dépôt: **13.09.1994**

(54) **Composition cosmétique contenant au moins un agent tensio-actif non-ionique du type alkylpolyglycoside et/ou polyglycérolé et au moins un copolymère séquencé polydiméthylsiloxane/polyoxyalkylène**

Kosmetisches Mittel enthaltend mindestens ein nichtionisches Tensid vom Typ Alkylpolyglykosid und/oder polyglyceroliert und mindestens ein Polydimethylsiloxan/Polyoxyalkylen Block-Copolymer

Cosmetic composition containing at least a nonionic tensid from the type alkylpolyglycosid and/or polyglycerolated and at least a polydimethylsiloxane/polyoxyalkylene bloc-copolymer

(84) Etats contractants désignés:
**DE ES FR GB IT**

(30) Priorité: **14.09.1993 FR 9310939**

(43) Date de publication de la demande:
**22.03.1995 Bulletin 1995/12**

(73) Titulaire: **L'OREAL**
**75008 Paris (FR)**

(72) Inventeurs:
- **Cauwet, Danièle**
  **F-75011 Paris (FR)**
- **Dubief, Claude**
  **F-78150 Le Chesnay (FR)**

(74) Mandataire: **Casalonga, Axel**
**BUREAU D.A. CASALONGA - JOSSE**
**Morassistrasse 8**
**80469 München (DE)**

(56) Documents cités:
**EP-A- 0 398 177**
**EP-A- 0 440 542**
**EP-A- 0 492 657**
**EP-A- 0 535 367**
**WO-A-92/08439**
**GB-A- 2 242 129**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 643 961 B1

Printed by Rank Xerox (UK) Business Services
2.14.18/3.4

## Description

L'invention concerne des compositions cosmétiques contenant au moins un agent tensio-actif non-ionique du type alkylpolyglycoside et/ou polyglycérolé et au moins un copolymère séquencé polydiméthylsiloxane/polyoxyalkylène.

Les copolymères séquencés polydiméthylsiloxane/polyoxyalkylène sont connus comme agents de conditionnement dans les compositions cosmétiques pour le traitement de la peau ou des cheveux. ns sont décrits dans la demande de brevet EP-0492657.

Les agents tensio-actifs de la famille des alkylpolyglycosides ou polyglycérolés ont déjà été préconisés dans des compositions lavantes pour les cheveux ou la peau. Ce sont des détergents doux, bien tolérés et biodégradables.

Les cheveux, agressés par les agents atmosphériques tels que la lumière ou les traitements chimiques, et lavés avec des bases lavantes classiques, sont difficilement démêlables et cet inconvénient se trouve encore accentué dans le cas de cheveux fins.

Les compositions de shampooing ne contenant que des tensioactifs non-ioniques tels que ceux cités ci-dessus, ne conduisent pas à de bonnes propriétés cosmétiques, en particulier le démêlage des cheveux mouillés est difficile et les cheveux à l'état sec sont rêches.

La demanderesse vient de découvrir de façon surprenante que l'association dans des compositions lavantes et/ou traitantes pour matières kératiniques, de copolymères séquencés polydiméthylsiloxane/polyoxyalkylène à des tensio-actifs non-ioniques particuliers du type alkylpolyglycoside et/ou du type polyglycérolé, conférait à ces compositions des propriétés de démêlage des cheveux mouillés, de lissage et de mise en place des cheveux considérablement améliorées.

En outre, la demanderesse a constaté qu'une telle association conférait aux cheveux une plus grande douceur à l'état sec.

D'autre part, les compositions contenant cette association apportent une plus grande facilité de coiffage.

La présente invention a donc pour objet des compositions cosmétiques contenant au moins un agent tensio-actif non-ionique du type alkylpolyglycoside et/ou polyglycérolé et au moins un copolymère séquencé polydiméthylsiloxane/polyoxyalkylène.

Un autre objet de l'invention est constitué par l'utilisation de ces compositions pour le traitement et/ou le lavage des matières kératiniques telles que la peau ou les cheveux.

Un autre objet de l'invention concerne un procédé de traitement cosmétique des cheveux et/ou de la peau mettant en oeuvre ces compositions.

D'autres objets de l'invention apparaîtront à la lumière de la description et des exemples qui suivent.

Les compositions cosmétiques selon l'invention contiennent dans un milieu aqueux cosmétiquement acceptable, au moins un agent tensio-actif non-ionique du type alkylpolyglycoside et/ou polyglycérolé et au moins un copolymère séquencé polydiméthylsiloxane/polyoxyalkylène.

Les copolymères polydiméthylsiloxane/polyoxyalkylène séquencés conformes à la présente invention, sont choisis de préférence parmi ceux répondant à la formule :

$$([Y(R_2SiO)_aR_2SiYO][(C_nH_{2n}O)_b])_c \qquad (I)$$

dans laquelle :

- R représente un radical hydrocarboné monovalent ne contenant pratiquement pas de groupement aliphatique insaturé;
- n vaut 2, 3 ou 4;
- a est un entier supérieur ou égal à 5;
- b est un entier supérieur ou égal à 4;
- c est un nombre supérieur ou égal à 4;
- Y représente un groupe organique divalent lié à un atome de silicium adjacent par une liaison Si-C et à un bloc polyoxyalkylène par un atome d'oxygène.

Les copolymères de formule (I) ont une masse moléculaire moyenne, de préférence supérieure ou égale à environ 3000. La masse moléculaire de chaque bloc siloxane est comprise entre environ 300 et 10.000; la masse moléculaire de chaque bloc polyoxyalkylène est comprise entre environ 300 et 10.000; la proportion en blocs de siloxane est comprise entre environ 10 et 95% en poids par rapport au poids total du copolymère séquencé.

Les radicaux R sont choisis notamment parmi les radicaux alkyle tels que méthyle, éthyle, propyle, isopropyle, butyle, pentyle, hexyle, octyle, décyle, dodécyle, octadécyle, eicosyle; les radicaux aryle tels que phényle et naphtyle; les radicaux aralkyle tels que benzyle et phényléthyle; les radicaux tolyle, xylyle ou cyclohexyle. Ils peuvent être identiques ou différents.

Parmi les groupes divalents organiques Y, on peut citer les groupes de structure :

-R'-, -R'-CO-, -R'-NHCO-, -R'-NHCONH-R"-NHCO-, -R'-OCONH-R"-NHCO-
où R' est un groupe alkylène divalent tel que éthylène, propylène, butylène et R" est identique à R' ou un groupe arylène tel que : $-C_6H_4-$, $-C_6H_4-C_6-H_4$, $-C_6H_4-CH_2-C_6H_4-$, $-C_6H_4-CH(CH_3)_2-C_6-H_4-$ et de préférence phénylène.

Les groupes divalents organiques préférentiels sont choisis parmi :
$-CH_2-CH_2-$, $-C_3H_6-$, $-C_4C_8-$, $-(CH_2)_2-CO-$, $-(CH_2)_3-NHCO-$, $-(CH_2)_3-NHCONHC_6H_4NHCO-$ et $-(CH_2)_3-OCONHC_6H_4NHCO-$

Les copolymères conformes à la présente invention sont connus et décrits dans la demande EP-0492657.

Les copolymères séquencés polydiméthylsiloxane/polyoxyalkylène particulièrement préférés selon l'invention, sont choisis parmi ceux de formule (IA) suivante :

$$[C_4H_8O(C_nH_{2n}O)_bC_4H_8SiMe_2O(\ SiMe_2O)_aSiMe_2]_c \qquad (IA)$$

où Me représente méthyle; n est un entier de 2 à 4; a et b sont des entiers d'au moins 4, et c est un nombre d'au moins 4.

Parmi ces copolymères, on utilise plus particulièrement ceux ayant un motif récurant de formule :

$$[\!-(Si\text{-}Me_2O]_x\text{-}\ Si\text{-}Me_2\text{-}\ C_4H_8O\text{-}\ (C_2H_4O)_y\text{-}\ (C_3H_6O)_z\text{-}\ C_4H_8]\!-$$

où x est un nombre compris entre 5 et 15 inclus; y est un nombre compris entre 15 et 30 inclus, et z est un nombre compris entre 20 et 40 inclus.

Les copolymères séquencés polydiméthylsiloxane/polyoxyalkylène sont présents dans les compositions dans des proportions comprises entre 0,1 et 5% en poids et de préférence entre 0,5 et 3% en poids par rapport au poids total de la composition.

Les alkylpolyglycosides utilisables conformément à l'invention répondent en particulier à la formule (II) suivante :

$$R(C_6H_{10}O_5)_x\text{-}H \qquad (II)$$

où

R est défini par :

$$R_1\text{-}O\text{-}(R_2O)_m\text{ -}$$

avec
$R_1$ représentant un groupement alkyle ramifié ou linéaire, ayant de 8 à 18 atomes de carbone, un groupement alcényle ramifié ou linéaire ayant de 8 à 18 atomes de carbone ou un groupement alkylphényle ayant de 8 à 18 atomes de carbone et où la partie alkyle est soit linéaire, soit ramifiée, et
$R_2$ représente un groupe alkylène ayant de 2 à 4 atomes de carbone,
et avec m compris entre 0 et 10.

Les composés préférés sont ceux où $R_1$ est un groupement alkyle ayant de 8 à 18 atomes de carbone, et de préférence de 10 à 14, tels que les groupements décyle, lauryle, myristyle.

La valeur de m préférentielle est comprise entre 0 et 3, et la valeur la plus préférentielle est 0;
x est un nombre compris entre 1 et 10.

Les composés alkylpolyglycosides de formule développée (IV) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; PLANTAREN 1300, 1200 UP, 2000 UP; les produits vendus par la Société SEPPIC sous les dénominations TRITON CG 110 (ou ORAMIX CG 110) et TRITON CG 312 (ou ORAMIX NS 10); ceux vendus par la Société BASF sous la dénomination LUTENSOL GD 70.

Les agents tensio-actifs non-ioniques du type polyglycérolé utilisés conformément à la présente invention, sont choisis de préférence parmi les composés polyhydroxypropyléthers suivants :

(A) Les composés répondant à la formule (IV) :

$$RO[(C_3H_5(OH)]\!-_nH \qquad (IV)$$

dans laquelle le groupement $[C_3H_5\ (OH)]$ représente les structures suivantes, prises ensemble ou séparément :

$$[\!-CH_2\ CH\ OH\ \text{-}\ CH_2\ \text{-}\ O]\!- \qquad (IVa)$$

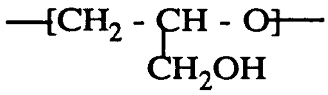 (IVb) et 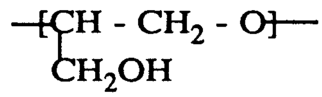 (IVc)

et R et n ont une des significations ci-après :

a) R représente un radical ou un mélange de radicaux alkyle en $C_{10}$-$C_{14}$ et n est un nombre entier ou décimal de 2 à 10 et de préférence 3 à 6.
b) R représente un reste :

$$R_2\ CONH\ CH_2 - CH_2O\ CH_2 - CH_2 - \qquad (V)$$

où $R_2$ désigne un radical ou un mélange de radicaux alkyle et/ou alcényle en $C_{11}$-$C_{17}$ et n désigne un nombre entier ou décimal de 1 à 5 et de préférence de 1,5 à 4.
c) R représente un reste :

$$R_3 - CHOH - CH_2 - \qquad (VI)$$

où $R_3$ désigne un radical aliphatique, cycloaliphatique, arylaliphatique en $C_7$-$C_{21}$ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.

Ces tensioactifs de formule (IV) peuvent être préparés selon les procédés décrits dans les brevets FR 1 477 048, 2 328 763 et 2 091 516;
(B) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol. Le procédé de préparation de ces composés est décrit dans le brevet FR-A-2 169 787;
(C) Les composés polyhydroxypropyléthers préparés par polyaddition de monochlorhydrine du glycérol sur un composé organique polyhydroxylé en présence d'une base forte, avec élimination au fur et à mesure de l'eau par distillation. Ces composés sont décrits dans le brevet français FR-A-2 574 786.

Parmi les tensio-actifs non ioniques de la famille des polyhydroxypropyléthers décrits dans les paragraphes (A), (B) et (C) ci-dessus, les composés préférés sont représentés par les formules:

(α)

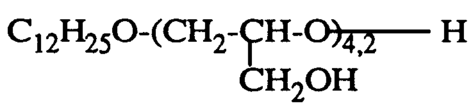 (VII)

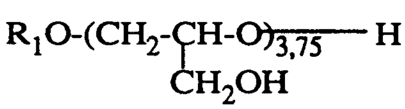 (VIII)

où $R_1$ désigne un mélange de radicaux alkyles en $C_{10}H_{21}$ et $C_{12}H_{25}$;
(β) les composés préparés par condensation en catalyse alcaline, de 3,5 moles glycidol sur un alphadiol ayant 12 atomes de carbone, selon le procédé décrit dans le brevet FR-A-2 091 516;
(γ) les composés répondant à la formule :

$$R_2\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{3,5}H \qquad (IX)$$

où $R_2$ désigne un mélange de radicaux comprenant les radicaux alkyle et alcényle suivants : $C_{11}H_{23}$,$C_{13}H_{27}$, les

radicaux dérivés des acides gras du coprah et le radical dérivé de l'acide oléique;

(δ) les composés préparés par condensation de 3,5 moles de glycidol sur un mélange d'alphadiols en $C_{11}$-$C_{14}$, décrits dans le brevet FR-A-2 091 516.

Le tensio-actif non ionique polyhydroxypropyléther obtenu par condensation de la monochlorhydrine du glycérol (2,5 moles) en présence de soude sur le dodécanediol-1,2, est plus particulièrement préféré.

Si les compositions selon l'invention ne sont pas utilisées pour le lavage des matières kératiniques, le ou les agents tensio-actifs non ioniques de type alkylpolyglycoside et/ou polyglycérolé sont utilisés dans de telles compositions dans des proportions comprises de préférence entre 0,05 et 5 % en poids par rapport au poids total de la composition. Ces compositions sont utilisées notamment comme compositions à rincer, appliquées avant ou après un shampooing, une coloration, une décoloration, une permanente, un défrisage, comme composition de coloration ou de permanente, ou comme composition non rincée de coiffage, de soin, de protection.

Si les compositions selon l'invention sont des compositions lavantes, elles contiennent le ou les tensio-actifs non ioniques du type alkylpolyglycoside et/ou polyglycérolé dans des proportions comprises entre 1 et 30 % en poids par rapport au poids total de la composition et plus particulièrement entre 5 et 20 % en poids.

Le pH des compositions conforme à l'invention est généralement compris entre 2 et 10, plus particulièrement entre 4 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en $C_1$-$C_4$, comme l'éthanol, l'isopropanol, le n-butanol; les alkylèneglycols, comme le propylèneglycol, les éthers de glycols.

Les compositions selon l'invention peuvent se présenter sous forme de liquides plus ou moins épaissis, de gels, d'émulsions (laits ou crèmes), de lotions aqueuses ou hydroalcooliques, de dispersions, ou de mousses aérosol.

Les compositions sont par exemples des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, ou des cils ou sourcils, des fards et fonds de teint pour le visage, des vernis à ongles, des shampooings, des produits pour le bain ou la douche, des compositions à rincer ou non, à appliquer avant ou après un shampooing, une coloration, une décoloration, une permanente ou un défrisage comme composition de coloration.

Les compositions conformes à l'invention peuvent éventuellement contenir, en outre, divers additifs qui n'altèrent pas les propriétés des compositions, telles que des agents tensioactifs anioniques, cationiques, amphotères ou zwittérioniques, des agents tensioactifs non ioniques autres que ceux décrits précédemment, des agents acidifiants ou alcalinisants, des agents conservateurs, des agents épaississants, des agents de mise en suspension, des adoucissants, des filtres solaires, des parfums, des biocides, des antioxydants, des pigments, des colorants, des agents nacrants, des biocides, des repellents, des oxydants, des réducteurs, des hydratants, des vitamines, des conservateurs, des renforçateurs de mousses, des électrolytes, des céramides, des filtres UV, des antibactériens, des agents antipelliculaires, des agents antiséborrhéiques, des antiparasitaires ou d'autres adjuvants couramment utilisés en cosmétique.

Les compositions conformes à l'invention sont appliqués sur la peau ou les cheveux dans une quantité efficace cosmétiquement, fonction de la nature de la composition.

Une application particulière des compositions selon l'invention est l'application en tant que composition de lavage et de traitement cosmétique des matières kératiniques, de préférence de la peau et des cheveux et plus particulièrement en tant que shampooing. Dans ce cas-là, le shampooing est appliqué sur les cheveux humides ou secs dans des quantités efficaces pour les laver, cette application étant suivie d'un rinçage.

Les exemples suivants sont destinés à illustrer l'invention.

<u>EXEMPLE 1</u>

On prépare un shampooing de composition suivante :

| | |
|---|---|
| - Alkyl($C_8$-$C_{10}$)polyglycoside vendu en solution aqueuse à 60% de MA sous la dénomination "ORAMIX CG 110" par la Société SEPPIC | 18 g MA |
| - Copolymère bloc de formule : | |
| $[[(CH_3)_2SiO]x(CH_3)_2SiCH_2CH(CH_3)CH_2O(C_2H_4O)_y\text{-}(C_3H_6O)zCH_2CH(CH_3)CH_2]_n$ | |
| avec x=9 à 11, y=18 à 25, z=28 à 35, | |
| et n en solution dans l'éthanol à 70% de MA, | |
| et tel que la viscosité d'une solution de polymère à 10% dans l'éthanol soit d'environ 50 mPa.s. | 3 g MA |
| - Heptaméthylnonane vendu sous la dénomination "ARLAMOL HD" par la Société ICI | 3 g |
| - Dérivé de dioléate de polyéthylèneglycol (55 moles d'oxyde d'éthylène) et de propylèneglycol, vendu sous la dénomination "ANTIL 141 Liquide" par la Société GOLDSCHMIDT à 43,6% de MA | 2 g MA |
| - Parfum, conservateur qs | |
| - Hydroxyde de sodium qs pH=5,5 | |
| - Eau qsp | 100 g |

EXEMPLE 2

On prépare un shampooing de composition suivante :

| | |
|---|---|
| - Alkyl($C_9$-$C_{10}$-$C_{11}$/20-40-40)polyglycoside (1,4), vendu à 50% de MA sous la dénomination "APG 300" par la Société HENKEL | 15 g MA |
| - Uréthane polyéther vendu sous la dénomination "DAPRAL T212" par la Société AKZO | 3 g |
| - $[[(CH_3)_2SiO]x(CH_3)_2SiCH_2CH(CH_3)CH_2O(C_2H_4O)_y\text{-}(C_3H_6O)zCH_2CH(CH_3)CH_2]_n$ | |
| avec x=9 à 11, y=18 à 25, z=28 à 35, | |
| et n en solution dans l'éthanol à 70% de MA, | |
| et tel que la viscosité d'une solution de polymère à 10% dans l'éthanol soit d'environ 50 mPa.s. | 2,5 g MA |
| - Polymère d'hydroxyéthylcellulose et d'épichlorhydrine quaternisé avec la triméthylamine, vendu sous la dénomination "CELQUAT SC 240" par la Société NATIONAL STARCH | 1 g |
| - Parfum, conservateur qs | |
| - HCl qs pH=6,5 | |
| - Eau qsp | 100 g |

EXEMPLE 3

On prépare un après-shampooing à rincer de composition suivante :

| | |
|---|---|
| - Alkyl($C_9$-$C_{10}$-$C_{11}$/20-40-40)polyglycoside (1,4), vendu à 50% de MA sous la dénomination "APG 300" par la Société HENKEL | 1 g MA |
| - $[[(CH_3)_2SiO]x(CH_3)_2SiCH_2CH(CH_3)CH_2O(C_2H_4O)_y\text{-}(C_3H_6O)zCH_2CH(CH_3)CH_2]_n$ avec x=9 à 11, y=18 à 25, z=28 à 35, et n en solution dans l'éthanol à 70% de MA, et tel que la viscosité d'une solution de polymère à 10% dans l'éthanol soit d'environ 50 mPa.s. | 2,5 g MA |
| - Emulsion de polyacrylamide vendue sous la dénomination "SEPIGEL 305" par la Société SEPPIC | 0,25 g en polymère |
| - Parfum, conservateur qs | |
| - pH spontné = 4,3 | |
| - Eau qsp | 100 g |

Les marques commerciales mentionnées dans les exemples sont, selon la connaissance de la Demanderesse, des marques déposées.

## Revendications

1. Composition cosmétique caractérisée par le fait qu'elle contient dans un milieu aqueux cosmétiquement acceptable, au moins un agent tensio-actif non-ionique de la famille des alkylpolyglycosides et/ou du type polyglycérolé et au moins un copolymère séquencé polydiméthy-siloxane/polyoxyalkylène.

2. Composition selon la revendication 1, caractérisée par le fait que le copolymère séquencé polydiméthylsiloxane/polyoxyalkylène correspond à la formule suivante :

$$([Y(R_2SiO)_aR_2SiYO][(C_nH_{2n}O)_b])_c \quad (I)$$

dans laquelle :

R représente un radical hydrocarboné monovalent ne contenant pratiquement pas de groupement aliphatique insaturé;
n vaut 2, 3 ou 4;
a est un entier supérieur ou égal à 5;
b est un entier supérieur ou égal à 4;
c est un nombre supérieur ou égal à 4;
Y représente un groupe organique divalent lié à un atome de silicium adjacent par une liaison Si-C et à un bloc polyoxyalkylène par une liaison avec un atome d'oxygène.

3. Composition selon la revendication 2, caractérisée par le fait que le copolymère de formule (I) a une masse moléculaire moyenne supérieure ou égale à 3000; la masse moléculaire moyenne de chaque bloc polyoxyalkylène ou siloxane étant comprise entre 300 et 10.000 et la proportion en blocs siloxane étant comprise entre 10 et 95% en poids par rapport au poids total du copolymère séquencé.

4. Composition selon la revendication 2, caractérisée par le fait que, dans la formule (I) :

les radicaux R, identiques ou différents, désignent un radical alkyle, aryle, aralkyle, tolyle, xylyle ou cyclohexyle;
Y désigne un groupe choisi parmi :
-R'-, -R'-CO-, -R'-NHCO-, -R'-NHCONH-R''-NHCO-, -R'-OCONH-R''-NHCO-où
R' est un alkylène divalent; R'' est identique à R' ou désigne un groupe arylène.

5. Composition selon la revendication 4, caractérisée par le fait que dans la formule (I) :

les radicaux R sont choisis parmi méthyle, éthyle, propyle, isopropyle, butyle, pentyle, hexyle, octyle, décyle, dodécyle, octadécyle, eicosyle; phényle, naphtyle; benzyle, phényléthyle; tolyle, xylyle; cyclohexyle;
R' est choisi parmi éthylène, propylène et butylène et R" est choisi parmi :
$-C_6H_4-$, $-C_6H_4-C_6-H_4$, $-C_6H_4-CH_2-C_6H_4-$, $-C_6H_4-CH(CH_3)_2C_6H_4-$.

**6.** Composition selon l'une quelconque des revendications 1 à 5, caractérisée par le fait que le copolymère séquencé polydiméthylsiloxane/polyoxyalkylène correspond à la formule (IA) suivante :

$$[C_4H_8O(C_nH_{2n}O)_bC_4H_8SiMe_2O(SiMe_2O)_aSiMe_2]_c \quad \text{(IA)}$$

dans laquelle a est un entier supérieur ou égal à 4; b est un entier supérieur ou égal à 4; c est un nombre supérieur ou égal à 4 et n est un antier de 2 à 4; Me représente méthyle.

**7.** Composition selon la revendication 6, caractérisée par le fait que le copolymère séquencé polydiméthylsiloxane/polyoxyalkylène a pour motif récurant celui de structure :

$$\{(Si\text{-}Me_2O]_x\text{-}\ Si\text{-}Me_2\text{-}\ C_4H_8O\text{-}\ (C_2H_4O)_y\text{-}\ (C_3H_6O)_z\text{-}\ C_4H_8\ \}$$

dans laquelle x est compris entre 5 et 15 inclus; y est compris entre 15 et 30 inclus; z est compris entre 20 et 30 inclus; Me représente méthyle.

**8.** Composition selon l'une quelconque des revendications 1 à 7, caractérisée par le fait que le copolymère séquené polydiméthylsiloxane/polyoxyalkylène est présent dans des proportions comprises entre 0,1 et 5% en poids par rapport au poids total de la composition.

**9.** Composition selon l'une quelconque des revendications 1 à 8, caractérisée en ce que les agents tensio-actifs non-ioniques de la famille des alkylpolyglycosides répondent à la formule suivante (II) :

$$R(C_6H_{10}O_5)_x\text{-}H \quad \text{(II)}$$

où

R est défini par :

$$R_1\text{-}O\text{-}(R_2O)_m\text{ -}$$

avec
$R_1$ représentant un groupement alkyle ramifié ou linéaire, ayant de 8 à 18 atomes de carbone, un groupement alcényle ramifié ou linéaire ayant de 8 à 18 atomes de carbone ou un groupement alkylphényle ayant de 8 à 18 atomes de carbone et où la partie alkyle est soit linéaire, soit ramifiée, et
$R_2$ représente un groupe alkylène ayant de 2 à 4 atomes de carbone,
et avec m compris entre 0 et 10, et x désigne un nombre compris entre 1 et 10.

**10.** Composition selon l'une des revendications 1 à 8, caractérisée en ce que les agents tensioactifs non-ioniques polyglycérolés sont choisis parmi les polyhydroxypropyléthers suivants :

(A) Les composés répondant à la formule (IV) :

$$RO[(C_3H_5\ (OH)\}_nH \quad \text{(IV)}$$

dans laquelle le groupement $[C_3\text{-}H_5\ (OH)]$ représente les structures suivantes, prises ensemble ou séparément :

$$\{CH_2CHOH\ \text{-}\ CH_2\ \text{-}\ O\} \quad \text{(IVa),}$$

et

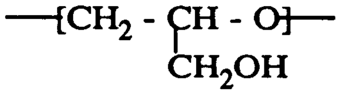 (IVb) et 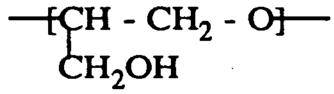 (IVc)

et R et n ont une des significations ci-après :

a) R représente un radical ou un mélange de radicaux alkyle en $C_{10}$-$C_{14}$ et n est un nombre entier ou décimal de 2 à 10 et de préférence de 3 à 6.
b) R représente un reste :

$$R_2\ CONH\ CH_2 - CH_2OCH_2 - CH_2 - \qquad (V)$$

où $R_2$ désigne un radical ou un mélange de radicaux alkyle et/ou alcényle en $C_{11}$-$C_{17}$ et n désigne un nombre entier ou décimal de 1 à 5 et de préférence de 1,5 à 4.
c) R représente un reste :

$$R_3 - CHOH - CH_2 - \qquad (VI)$$

où $R_3$ désigne un radical aliphatique, cycloaliphatique, arylaliphatique en $C_7$-$C_{21}$ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.

(B) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol.
(C) Les composés polyhydroxypropyléthers préparés par polyaddition de monochlorhydrine du glycérol sur un composé organique polyhydroxylé en présence d'une base forte, avec élimination au fur et à mesure de l'eau par distillation.

**11.** Composition selon la revendication 10, caractérisée en ce que les agents tensioactifs non-ioniques polyglycérolés sont choisis parmi les polyhydroxypropyléthers suivants :

(α)

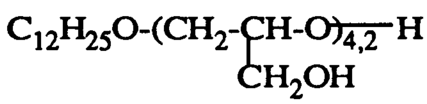 (VII)

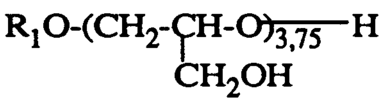 (VIII)

où $R_1$ désigne un mélange de radicaux alkyles en $C_{10}H_{21}$ et $C_{12}\ H_{25}$;
(β) les composés préparés par condensation en catalyse alcaline, de 3,5 moles de glycidol sur un alphadiol ayant 12 atomes de carbone.
(γ) les composés répondant à la formule :

$$R_2\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{3,5}\ H \qquad (IX)$$

où $R_2$ désigne un mélange de radicaux comprenant les radicaux alkyle et alcényle suivants : $C_{11}H_{23}$, $C_{13}H_{27}$, les radicaux dérivés des acides gras du coprah et le radical dérivé de l'acide oléique;
(δ) les composés préparés par condensation de 3,5 moles de glycidol sur un mélange d'alphadiols en $C_{11}$-$C_{14}$ ;

**12.** Composition selon l'une quelconque des revendications 1 à 11, caractérisée en ce qu'elle renferme 0,05 à 5 % en poids par rapport au poids total de la composition, d'agents tensio-actifs non-ioniques de la famille des alkylpolyglycosides et/ou polyglycérolés.

13. Composition selon l'une quelconque des revendications 1 à 11, caractérisée en ce qu'elle renferme 1 à 30 % en poids, de préférence 5 à 20 % en poids par rapport au poids total de la composition, d'agents tensio-actifs non-ioniques de la famille des alkylpolyglycosides et/ou des polyglycérolés.

14. Composition selon l'une quelconque des revendications 1 à 13, caractérisée en ce que le milieu aqueux cosmétiquement acceptable est constitué uniquement par de l'eau ou par un mélange d'eau et d'un solvant cosmétiquement acceptable.

15. Composition selon l'une quelconque des revendications 1 à 14, caractérisée en ce qu'elle se présente sous forme de liquide plus ou moins épaissi, de gel, d'émulsion, de lotion aqueuse ou hydroalcoolique, de dispersion, ou de mousse aérosol.

16. Composition selon l'une quelconque des revendications 1 à 15, caractérisée en ce qu'elle contient des agents tensio-actifs anioniques, cationiques, amphotères ou zwitterioniques, d'autres agents tensioactifs non-ioniques, des protéines, des agents acidifiants ou alcalinisants, des agents conservateurs, des agents épaississants, des agents de mise en suspension, des adoucissants, des filtres solaires, des parfums, des biocides, des antioxydants, des pigments, des colorants, des agents nacrants, des biocides, des repellents, des oxydants, des réducteurs, des hydratants, des vitamines, des conservateurs, des renforçateurs de mousses, des électrolytes, des céramides, des filtres UV, des antibactériens, des agents antipelliculaires, des agents antiséborrhéiques, des antiparasitaires ou d'autres adjuvants couramment utilisés en cosmétique.

17. Utilisation de la composition telle que définie dans l'une quelconque des revendications 1 à 16, pour le traitement et/ou le lavage de matières kératiniques constituées par les cheveux, la peau, les cils ou les sourcils.

18. Procédé de traitement cosmétique, caractérisé en ce qu'il consiste à appliquer sur la peau ou les cheveux, une quantité cosmétiquement efficace d'une composition selon l'une quelconque des revendications 1 à 17 .

19. Procédé de lavage et de traitement cosmétique des cheveux, caractérisé en ce qu'il consiste à appliquer sur les cheveux humides ou secs, une quantité efficace pour les laver, de la composition telle que décrite dans l'une quelconque des revendications 1 à 11 et 13 à 16, et qu'on procède ensuite au rinçage à l'eau.

**Claims**

1. A cosmetic composition characterized by the fact that it contains, in a cosmetically acceptable aqueous medium, at least one non- ionic surfactant of the alkylpolyglycoside family and/or of the polyglycerol type and at least one sequenced polydimethylsiloxane/polyoxyalkylene copolymer.

2. The composition of Claim 1, characterized by the fact that the sequenced polydimethylsiloxane/polyoxyalkylene copolymer corresponds to the following formula:

$$([Y(R_2SiO)_aR_2SiYO][(C_nH_{2n}O)_b])_c \qquad (I)$$

where:

R is a monovalent hydrocarbon radical containing practically no unsaturated aliphatic groups,
n is 2, 3 or 4, a is an integer not less than 5,
b is an integer not less than 4,
c is a number not less than 4,
Y is a divalent organic group bound to an adjacent silicon atom by an Si/C bond and to a polyoxyalkylene block by an oxygen atom.

3. The composition of Claim 2, characterized by the fact that the copolymer with formula (I) has an average molecular weight greater than or equal to 3000. The average molecular weight of each polyalkylene or siloxane block is between 300 and 10,000, and the proportion of siloxane blocks is between 10 and 95% by weight of the total weight of the sequenced copolymer.

4. The composition of Claim 2, characterized by the fact that, in formula (I):

the radicals R, identical or different, denote an alkyl, aryl, aralkyl, tolyl, xylyl or cyclohexyl radical,

Y denotes a group selected from among:
-R'-, -R'-CO-, -R'-NHCO-, -R'-NHCONH-R"-NHCO-, -R'-OCONH-R"-NHCO-where
R' is a divalent alkylene, and R" is identical to R' or denotes an arylene group.

**5.** The composition of Claim 4, characterized by the fact that, in the formula (I):

the R radicals are selected from among methyl, ethyl, propyl, isopropyl, butyl, pentyl, hexyl, octyl, decyl, dodecyl, octadecyl, eicosyl, or phenyl, naphthyl, or benzyl, phenylethyl, or tolyl, xylyl, or cyclohexyl,
R' is selected from among ethylene, propylene and butylene, and R" is selected from among:
$-C_6H_4-$, $-C_6H_4-C_6-H_4$, $-C_6H_4-CH_2-C_6H_4-$, $-C_6H_4-CH(CH_3)_2-C_6H_4-$ .

**6.** The composition of any of Claims 1 to 5, characterized by the fact that the sequenced polydimethylsiloxane/polyoxyalkylene copolymer corresponds to the following formula (IA):

$$[C_4H_8O(C_nH_{2n}O)_bC_4H_8SiMe_2O(SiMe_2O)_aSiMe_2]_c \qquad \text{(IA)}$$

where a is an integer greater than or equal to 4, b is an integer greater than or equal to 4, c is a number greater than or equal to 4, and n is an integer from 2 to 4; Me represents methyl.

**7.** The composition of Claim 6, characterized by the fact that the sequenced polydimethylsiloxane/polyoxyalkylene copolymer has a recurrent motif with the following structure:

$$\{(SiMe_2O]_x\text{-} Si\text{-}Me_2\text{-} C_4H_8O\text{-} (C_2H_4O)_y\text{-} (C_3H_6O)_z\text{-} C_4H_8\}$$

in which x is between 5 and 15 inclusive, y is between 15 and 30 inclusive, and z is between 20 and 30 inclusive; Me represents methyl.

**8.** The composition of any one of Claims 1 to 7, characterized by the fact that the sequenced polydimethylsiloxane/polyoxyalkylene copolymer is present in proportions of between 0.1 and 5% by weight of the total weight of the composition.

**9.** The composition of any one of Claims 1 to 8, characterized in that the non-ionic surfactants of the alkylpolyglycoside family answer to the following formula (II):

$$R(C_6H_{10}O_5)_x\text{-}H \qquad \text{(II)}$$

where R is defined by:

$$R_1\text{-}O\text{-}(R_2O)_m\text{ -}$$

where R1 is a branched or linear alkyl group, having 8 to 18 carbon atoms, a branched or linear alkenyl group having 8 to 18 carbon atoms, or an alkylphenyl group having 8 to 18 carbon atoms, and where the alkyl portion is either linear or branched, and R2 is an alkylene group having 2 to 4 carbon atoms, and where m is between 0 and 10, and x denotes a number between 1 and 10.

**10.** The composition of any of Claims 1 to 8, characterized in that the polyglycerol non-ionic surfactants are selected from among the following polyhydroxypropylethers:

(A) Compounds answering to the formula (IV):

$$RO\ [(C_3H_5\ (OH)\}_n\ H \qquad \text{(IV)}$$

in which the $[C_3H_5(OH)]$ group represents the following structures, used together or separately:

$$\{CH_2\ CH\ OH\ \text{-}\ CH_2\ \text{-}\ O\} \qquad \text{(IVa)}$$

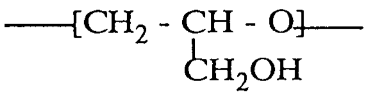 (IVb) and 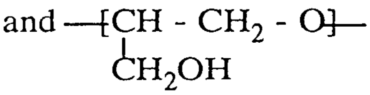 (IVc)

and R and n have the following meanings:

(a) R is a radical or a mixture of $C_{10}$-$C_{14}$ alkyl radicals and n is a whole or decimal number from 2 to 10 and preferably from 3 to 6.
(b) R is a remnant:

$$R_2\ CONH\ CH_2 - CH_2OCH_2 - CH_2 - \qquad (V)$$

where $R_2$ denotes a radical or a mixture of alkyl and/or alkenyl radicals in $C_{11}$-$C_{17}$ and n denotes a whole or decimal number from 1 to 5 and preferably from 1.5 to 4;
(c) R is a remnant:

$$R_3 - CHOH - CH_2 - \qquad (VI)$$

where R3 denotes an aliphatic, cycloaliphatic, arylaliphatic radical in $C_7$-$C_{21}$ and their mixtures, the aliphatic chains denoting in particular the alkyl chains having 1 to 6 ether, thioether and/or hydroxymethylene groups, and n denotes a whole or decimal number from 1 to 10;

(B) The compounds prepared by condensation, in acidic catalysis, of 2 to 10 and preferably of 2.5 to 6 moles of glycidol per mole of alcohol or alpha-diol containing 10 to 14 carbon atoms, at a temperature from 50 to 120 °C, the glycidol being added slowly to the alcohol or to the alpha-diol;
(C) The polyhydroxypropylether compounds prepared by polyaddition of glycerol monochlorhydrin to a polyhydroxyl organic compound in the presence of a strong base, with progressive removal of water by distillation.

**11.** The composition of Claim 10, characterized in that the polyglycerol non-ionic surfactants are selected from among the follo wing polyhydroxypropylethers:

(α)

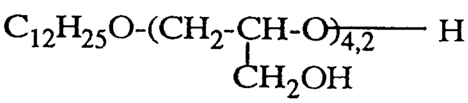 (VII)

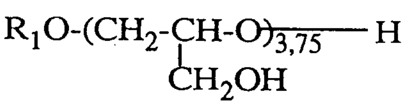 (VIII)

where R1 denotes a mixture of alkyl radicals in C10H21 and C12H25;
(β) The compounds prepared by condensation in alkaline catalysis, of 3.5 moles of glycidol on an alpha-diol having 12 carbon atoms;
(γ) The compounds answering to the formula:

$$R_2\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{3,5}\ H \qquad (IX)$$

where R2 denotes a mixture of radicals containing the following alkyl and alkenyl radicals:
$C_{11}H_{23}H_{27}$, the radicals derived from fatty acids of copra and the radical derived from oleic acid;
(δ) The compounds prepared by condensation of 3.5 moles of glycidol on a mixture of alpha-diols in $C_{11}$-$C_{14}$.

**12.** The composition of any one of Claims 1 to 11, characterized in that it contains 0.5 to 5% by weight of the total weight of the composition of non-ionic surfactants of the alkylpolyglycoside and/or polyglycerol family.

13. The composition of any one of Claims 1 to 11, characterized in that it contains 1 to 30% by weight, and preferably 5 to 20% by weight of the total weight of the composition, of non-ionic surfactants of the alkylpolyglycoside and/or polyglycerol family.

14. The composition of any one of Claims 1 to 13, characterized in that the cosmetically acceptable aqueous medium consists exclusively of water or of a mixture of water and a cosmetically acceptable solvent.

15. The composition of any one of Claims 1 to 14, characterized in that it is present in the form of a liquid of varying consistency, gel, emulsion, aqueous or hydro-alcoholic lotion, dispersion, or aerosol foam.

16. The composition of any one of Claims 1 to 15, characterized in that it contains anionic, cationic, amphoteric or zwitterionic surfactants, non-ionic surfactants other than those described previously, acidizing or alkalinizing agents, preservatives, thickeners, suspending agents, softeners, sun filters, perfumes, biocides, anti- oxidants, pigments, dyes, pearl lustering agents, biocides, repellants, oxidants, reducing agents, moisturizers, vitamins, preservatives, foam reinforcers, electrolytes, ceramides, UV filters, anti-bacterial agents, anti- dandruff agents, anti-seborrhoeic agents, pesticides, or other additives routinely used in cosmetics.

17. Use of the composition as defined in any one of Claims 1 to 16, for the treatment and/or washing of keratinic materials consisting of the hair, the skin, the eyelashes or the eyebrows.

18. A process of cosmetic treatment, characterized in that it consists in applying to the skin or the hair a cosmetically effective quantity of a composition according to any one of Claims 1 to 17.

19. A process of washing and cosmetic treatment of the hair, characterized in that it consists in applying to the wet or dry hair an effective quantity to wash it, of the composition as described in any one of Claims 1 to 11 and 13 to 16, and further characterized in that this is followed by rinsing with water.

**Patentansprüche**

1. Kosmetische Zusammensetzung
dadurch **gekennzeichnet**, daß sie in einem kosmetisch geeigneten wässrigen Milieu mindestens ein nicht-ionisches oberflächenaktives Mittel der Familie der Alkylpolyglycoside und/oder des polyglycerierten Typs und mindestens ein Polydimethylsiloxan/Polyoxalkylen-Sequenz-Copolymer enthält.

2. Zusammensetzung gemäß Anspruch 1,
dadurch **gekennzeichnet**, daß das Polydimethylsiloxan/Polyoxalkylen-Sequenz-Copolymer die folgende Formel aufweist:

$$([Y(R_2SiO)_aR_2SiYO][(C_nH_{2n}O)_b])_c \qquad (I)$$

worin gilt:

R stellt einen einwertigen Kohlenwasserstoffrest dar, der praktisch keine ungesättigte aliphatische Gruppierung aufweist;
n beträgt 2, 3 oder 4;
a ist eine ganze Zahl größer oder gleich 5;
b ist eine ganze Zahl größer oder gleich 4;
c ist eine Zahl größer oder gleich 4;
Y stellt eine zweiwertige organische Gruppe dar, die an ein benachbartes Siliziumatom über eine Si-C-Bindung und an einen Polyoxalkylen-Block über eine Bindung mit einem Sauerstoffatom gebunden ist,

3. Zusammensetzung gemäß Anspruch 3, dadurch **gekennzeichnet**, daß
das Copolymer der Formel (I) eine mittlere Molekularmasse von größer oder gleich 3000 aufweist, die mittlere Molekularmasse eines jeden Polyoxalkylen- oder Siloxan-Blocks 300 bis 10000 und der Anteil an Siloxan-Blöcken 10 bis 95 Gew.% betragen, bezogen auf das Gesamtgewicht des Sequenz-Copolymer.

4. Zusammensetzung gemäß Anspruch 2,
dadurch **gekennzeichnet**, daß in der Formel (I) gilt:

die Reste R stellen, gleich oder verschieden, einen Alkyl-, Aryl-, Aralkyl-, Tolyl-, Xylyl- oder Cyclohexylrest dar;

Y bedeutet eine Gruppe, ausgewählt aus:
-R'-, -R'-CO-, -R'-NHCO-, -R'-NHCONH-R"-NHCO-, -R'-OCONH-R"-NHCO-,
worin R' ein zweiwertige Alkylenrest ist und R" identisch mit R' ist oder eine Arylengruppe bedeutet.

**5.** Zusammensetzung gemäß Anspruch 4,
dadurch **gekennzeichnet**, daß
in der Formel (I) gilt:
die Reste R sind aus Methyl-, Ethyl-, Propyl-, Isopropyl-, Butyl-, Pentyl-, Hexyl-, Octyl-, Decyl-, Dodecyl-, Octadecyl-, Eicosyl-, Phenyl-, Naphthyl-, Benzyl-, Phenylethyl-, Tolyl-, Xylyl- und Cyclohexylresten ausgewählt;
R' ist aus Ethylen-, Propylen- und Butylenresten ausgewählt, und R" ist ausgewählt aus :
$-C_6H_4-$, $-C_6H_4-C_6H_4$, $-C_6H_4-CH_2-C_6H_4-$, $-C_6H_4-CH(CH_3)-C_6H_4-$.

**6.** Zusammensetzung gemäß einem der Ansprüche 1 bis 5,
dadurch **gekennzeichnet**, daß
das Polydimethylsiloxan/Polyoxalkylen-Sequenz-Copolymer die folgende Formel (IA) aufweist:

$$[C_4H_8O(C_nH_{2n}O)_bC_4H_8SiMe_2O(\ SiMe_2O)_aSiMe_2]_c \qquad (IA)$$

worin a eine ganze Zahl größer oder gleich 4, b eine ganze Zahl größer oder gleich 4, c eine Zahl größer oder gleich 4 und n eine ganze Zahl von 2 bis 4 sind und Me den Methylrest darstellt.

**7.** Zusammensetzung gemäß Anspruch 6,
dadurch **gekennzeichnet**, daß
das Polydimethylsiloxan/Polyoxalkylen-Sequenz-Copolymer als wiederkehrende Einheit diejenige der Struktur aufweist:

$$[(Si\text{-}Me_2O]_x\text{-}\ Si\text{-}Me_2\text{-}\ C_4H_8O\text{-}\ (C_2H_4O)_y\text{-}\ (C_3H_6O)_z\text{-}\ C_4H_8]$$

worin x 5 bis 15, y 15 bis 30 und z 20 bis 30 betragen und Me den Methylrest darstellt.

**8.** Zusammensetzung gemäß einem der Ansprüche 1 bis 7,
dadurch **gekennzeichnet**, daß
das Polydimethylsiloxan/Polyoxalkylen-Sequenz-Copolymer in Anteilen von 0,1 bis 5 Gew.% vorhanden ist, bezogen auf das Gesamtgewicht der Zusammensetzung.

**9.** Zusammensetzung gemäß einem der Ansprüche 1 bis 8,
dadurch **gekennzeichnet**, daß
die nicht-ionischen oberflächenaktiven Mittel der Familie der Alkylpolyglycoside die folgende Formel (II) aufweisen:

$$R(C_6H_{10}O_5)_x\text{-}H \qquad (II)$$

worin R definiert ist durch:

$$R_1\text{-}O\text{-}(R_2O)_m\text{-}$$

worin $R_1$ eine verzweigte oder lineare Alkylgruppe mit 8 bis 18 Kohlenstoffatomen, eine verzweigte oder lineare Alkenylgruppe mit 8 bis 18 Kohlenstoffatomen oder eine Alkylphenylgruppe mit 8 bis 18 Kohlenstoffatomen darstellt, worin der Alkylteil entweder linear oder verzweigt ist, und
$R_2$ eine Alkylengruppe mit 2 bis 4 Kohlenstoffatomen darstellt,
und worin m 0 bis 10 beträgt,
und worin x eine Zahl von 1 bis 10 bedeutet.

**10.** Zusammensetzung gemäß einem der Ansprüche 1 bis 8,
dadurch **gekennzeichnet**, daß
die nicht-ionischen polyglczerierten oberflächenaktiven Mittel aus den folgenden Polyhydroxipropylethern ausgewählt sind:

(A) Verbindungen der Formel (IV):

$$RO((C_3H_5(OH))_n\text{-}H \qquad (IV)$$

worin die Gruppierung ($C_3H_5(OH)$) die folgenden Strukturen darstellt, jeweils gemeinsam oder einzeln:

$$\{CH_2CHOH - CH_2 - O\} \qquad (IVa),$$

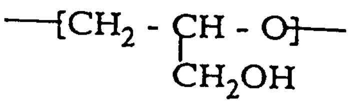 (IVb) und 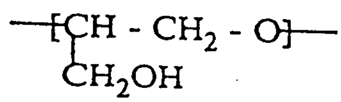 (IVc)

und worin R und n eine der folgenden Bedeutungen haben:

a) R stellt einen $C_{10\text{-}14}$-Alkylrest oder eine Mischung dieser Reste dar, und n ist eine ganze Zahl oder Dezimalzahl von 2 bis 10 und vorzugsweise von 3 bis 6;

b) R stellt einen Rest dar:

$$R_2CONHCH_2\text{-}CH_2OCH_2\text{-}CH_2\text{-} \qquad (V)$$

worin $R_2$ einen $C_{11\text{-}17}$-Alkyl- und/oder -Alkenylrest oder eine Mischung dieser Reste darstellt und n eine ganze Zahl oder Dezimalzahl von 1 bis 5 und vorzugsweise von 1,5 bis 4 bedeuten;

c) R stellt einen Rest dar:

$$R_3\text{-}CHOH\text{-}CH_2 \qquad (VI)$$

worin $R_3$ einen aliphatischen, cycloaliphatischen oder arylaliphatischen $C_{7\text{-}21}$-Rest und deren Mischungen, wobei die aliphatischen Ketten insbesondere Alkylketten bedeuten, die 1 bis 6 Ether-, Thioether- und/oder Hydroximethylen-Gruppierungen aufweisen können, und n eine ganze Zahl oder Dezimalzahl von 1 bis 10 bedeuten;

(B) aus Verbindungen, die durch Kondensation, unter saurer Katalyse, von 2 bis 10 und vorzugsweise von 2,5 bis 6 Mol Glycidol pro Mol Alkohol oder α-Diol mit 10 bis 14 Kohlenstoffatomen bei einer Temperatur von 50 bis 120°C hergestellt werden, wobei das Glycidol langsam zum Alkohol oder zum α-Diol gegeben wird;

(C) aus Polyhydroxipropylether-Verbindungen, die durch Polyaddition des Monochlorhydrin von Glycerin an eine polyhydroxylierte organische Verbindung in Gegenwart einer starken Base, unter Beseitigung des gebildeten Wassers durch Destillation, hergestellt werden.

**11.** Zusammensetzung gemäß Anspruch 10,
dadurch **gekennzeichnet**, daß
die nicht-ionischen polyglycerierten oberflächenaktiven Mittel aus den folgenden Polyhydroxipropylethern ausgewählt sind:

(α)

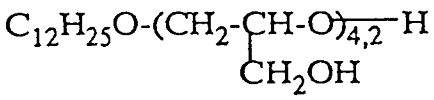

(VII)

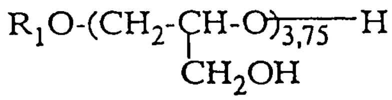

(VIII)

worin $R_1$ eine Mischung aus $C_{10}H_{21}$- und $C_{12}H_{25}$-Alkylresten bedeutet;

(β) durch Verbindungen, die durch Kondensation unter alkalischer Katalyse von 3,5 Mol Glycidol an ein α-Diol mit 12 Kohlenstoffatomen hergestellt sind;

(γ) durch Verbindungen der Formel:

$$R_2\text{-CONH-}CH_2\text{-}CH_2\text{-O-}CH_2\text{-}CH_2\text{-O-(}CH_2\text{-CHOH-}CH_2\text{-O)}_{3,5}\text{H} \quad \text{(IX)}$$

worin $R_2$ eine Mischung aus Resten bedeutet, die die folgenden Alkyl- und Alkenylreste umfassen: $C_{11}H_{23}$, $C_{13}H_{27}$, Reste, die aus Fettsäuren von Koprah abgeleitet sind, und den von Ölsäure abgeleiteten Rest;

(δ) aus Verbindungen, die durch Kondensation von 3,5 Mol Glycidol mit einer Mischung aus $C_{11\text{-}14}$-α-Diolen hergestellt sind.

**12.** Zusammensetzung gemäß einem der Ansprüche 1 bis 11, dadurch **gekennzeichnet**, daß sie 0,05 bis 5 Gew.%, bezogen auf das Gesamtgewicht der Zusammensetzung, nicht-ionische oberflächenaktive Mittel der Familie der Alkylpolyglycoside und/oder polyglycerierter Verbindungen enthält.

**13.** Zusammensetzung gemäß einem der Ansprüche 1 bis 11, dadurch **gekennzeichnet**, daß sie 1 bis 30 und vorzugsweise 5 bis 20 Gew.%, bezogen auf das Gesamtgewicht der Zusammensetzung, nicht-ionischer oberflächenaktiver Mittel der Familie der Alkylpolyglycoside und/oder polyglycerierter Verbindungen enthält.

**14.** Zusammensetzung gemäß einem der Ansprüche 1 bis 13, dadurch **gekennzeichnet**, daß das kosmetisch geeignete wässrige Milieu alleiniglich aus Wasser oder aus einer Mischung aus Wasser und einem kosmetisch geeigneten Lösungsmittel zusammengesetzt ist.

**15.** Zusammensetzung gemäß einem der Ansprüche 1 bis 14, dadurch **gekennzeichnet**, daß sie in Form einer mehr oder weniger verdickten Flüssigkeit, eines Gels, einer Emulsion, einer wässrigen oder hydroalkoholischen Lotion, einer Dispersion oder eines Aerosol-Schaums vorliegt.

**16.** Zusammensetzung gemäß einem der Ansprüche 1 bis 15, dadurch **gekennzeichnet**, daß sie anionische, kationische, amphotere oder zwitterionische oberflächenaktive Mittel, weitere nicht-ionische oberflächenaktive Mittel, Proteine, sauer oder alkalisch stellende Mittel, Konservierungsmittel, Verdickungsmittel, Suspendiermittel, Weichmacher, Sonnenfilterstoffe, Parfüm-Produkte, Biozide, Antioxidantien, Pigmente, Farbstoffe, Perlmuttglanzmittel, biozide Mittel, Abschreckmittel, oxidierende und reduzierende Mittel, Hydratisiermittel, Vitamine, Konservierungsstoffe, Schaumverstärkungsmittel, Elektrolyte, Ceramide, UV-Filterstoffe, antibakterielle Mittel, Antischuppenmittel, antiseborrheische Mittel, Mittel gegen Parasiten oder weitere Hilfsstoffe enthält, die laufend in der Kosmetik eingesetzt werden.

**17.** Verwendung der in jedem der Ansprüche 1 bis 16 definierten Zusammensetzung zur Behandldung oder zum Waschen keratinischer Materien aus Haaren, Haut, Wimpern oder Augenbrauen.

18. Verfahren zur kosmetischen Behandlung,
dadurch **gekennzeichnet**, daß
es darauf beruht, daß man auf die Haut oder die Haare eine kosmetisch wirksame Menge einer Zusammensetzung gemäß jedem der Ansprüche 1 bis 17 aufbringt und einwirken läßt.

19. Verfahren zum Waschen und kosmetischen Behandeln der Haare,
dadurch **gekennzeichnet**, daß
es darauf beruht, daß man auf die feuchten oder trockenen Haare eine zu deren Wäsche wirksame Menge der in jedem der Ansprüche 1 bis 11 und 13 bis 16 beschriebenen Zusammensetzung aufbringt und dann eine Spülung mit Wasser durchführt.